# NEUE EUROPÄISCHE PATENTSCHRIFT

(11) **EP 1 239 862 B3**
(45) Veröffentlichungstag dieser Patentschrift: **07.09.2011**
(45) Hinweis auf die Patenterteilung: 06.07.2005
(21) Anmeldenummer: 00979624.4
(22) Anmeldetag: 25.11.2000
(51) Int. Cl.: A61K 31/70, A61K 31/205, A61K 31/195, A61P 35/00

(54) **PHARMAZEUTISCHE ZUBEREITUNG ENTHALTEND ZYTOSTATIKA UND ELEKTRONENAKZEPTOREN ZUR BEHANDLUNG VON KREBS**
PHARMACEUTICAL PREPARATION CONTAINING CYTOSTATIC AGENTS AND ELECTRON ACCEPTORS FOR TREATING TUMOR DISEASES
PREPARATION PHARMACEUTIQUE CONTENANT DES AGENTS CYTOSTATIQUES ET DES ACCEPTEURS D'ELECTRONS POUR TRAITER DES AFFECTIONS TUMORALES

(30) Priorität: 09.12.1999 DE 19959546
(43) Veröffentlichungstag der Anmeldung: 18.09.2002
(73) Patentinhaber: A. Nattermann & Cie. GmbH, 50829 Köln (DE)
(72) Erfinder: GHYCZY, Miklos, 50933 Köln (DE); HAGER, Jörg, 50827 Köln (DE); WENDEL, Armin, 50859 Köln (DE)
(74) Vertreter: Fischer, Hans-Jürgen
(86) Internationale Anmeldenummer: PCT/EP2000/011761
(87) Internationale Veröffentlichungsnummer: WO 2001/041747

(56) Entgegenhaltungen:
- WO-A-00/06134
- WO-A-00/12071
- RUSSO S ET AL: "Effects of S- adenosylmethionine (SAMe) on doxorubicin -induced cardiotoxicity in the rat." JOURNAL OF MEDICINE, (1994) 25 (1-2) 65-89. , XP001028103
- NAMER M. ET AL: "[Clinical study of ferrous betainate plus cyanocobalamin and intrinsic factor in patients receiving antineoplastic chemotherapy]. ESSAI THERAPEUTIQUE EVALUANT L'APPORT DE L'ERYTHROTON DANS LE CADRE D'UNE CHIMIOTHERAPIE ONCOSTATIQUE." MEDECINE INTERNE, (1982) 17/8-9 (284-286). CODEN: MEITAL, XP001028102
- GHYCZY M. ET AL: "Electrophilic methyl groups present in the diet ameliorate pathological states induced by reductive and oxidative stress: A hypothesis." BRITISH JOURNAL OF NUTRITION, (2001) 85/4 (409-414). , XP001027911
- WANG, SHUANG ET AL: "Mitomycin Betaines: Synthesis, Structure, and Solvolytic Reactivity" J. ORG. CHEM. (1996), 61(26), 9202-9206 , XP001027903

## Beschreibung

Die vorliegende Erfindung betrifft eine pharmazeutische Zubereitung enthaltend mindestens einen zytostatisch wirkenden Wirkstoff, wenigstens einen biologischen Elektronenakzeptor und die üblichen pharmazeutischen Zusätze, sowie deren Verwendung zur Behandlung von Tumorerkrankungen, insbesondere zur Krebsbehandlung.

Es sind eine Reihe von pharmazeutischen Zubereitungen zur Behandlung von Tumorerkrankungen bekannt, wobei sich diese bekannten und zum Teil auch bereits angewendeten Zytostatika dadurch unterscheiden, daß sie unterschiedliche zytostatisch aktive Wirkstoffe, wie insbesondere Taxan, Taxanderivate, Taxole, Chinone, Benzochinone, andere Chinone sowie Derivate und/oder Salze dieser Verbindungen aufweisen.

Die internationale Patentanmeldung WO00/06134 offenbart die Verwendung von Zytostatika zusammen mit L-Camitin-Derivaten der Formel 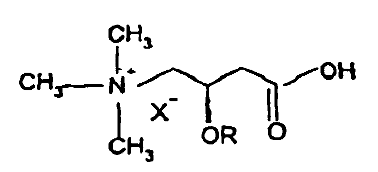 in der Krebstherapie.

Besonders erfolgversprechende Zytostatika stellen das 4H-1-Benzopyran-4-on und dessen Derivate dar, wobei diese Verbindungen auch Flavopiridole genannt werden und aus der europäischen Patentanmeldung 0 137 193, dem europäischen Patent 0 366 061 und der deutschen Offenlegungsschrift 36 12 337 bekannt sind. Insbesondere sind dies die Verbindungen, die in dem europäischen Patent 0 366 061 als Verbindungen der allgemeinen Formel B beschrieben sind, 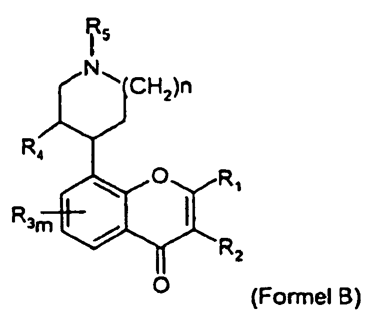 wobei
R₁ Wasserstoff, Alkyl mit 1 bis 6 Kohlenstoffatomen, Aryl-C₁-C₄-alkyl, substituiertes C₁-C₆-Alkyl, C₃-C₆-Cycloalkyl, einen C₃-C₉-Heterocyclus mit 1, 2 oder 3 Heteroatomen wie N, S, O oder deren beliebige Kombinationen, C₃-C₆-Cycloalkyl-C₁-C₄-alkyl, C₂-C₆-Alkenyl, C₂-C₆-Alkinyl, Aryl,polycyclische Ringe einbegriffen aromatische heterocyclische Reste, substituiertes Aryl, Carboxyl oder eine Aldehyd- oder COO-C₁-C₄-Alkylgruppe, eine primäre Amino-, Alkylamino-, Aralkylamino-, Dialkylamino-, Amido-, Arylamino-, Diarylaminogruppe oder -CH₂O-C₁-C₄-Alkyl bedeutet;
R₂ Wasserstoff oder C₁-C₃-Alkyl bedeutet;
R₃ Hydroxyl oder OCH₃ bedeutet;
R₄ Hydroxyl bedeutet;
R₅ CH₃ bedeutet;
m gleich der Zahl 2 ist und
n gleich der Zahl 1 ist,
und deren pharmakologisch unbedenkliche Säureadditionssalze.

Die erfindungsgemässen Verbindungen haben zwei asymmetrische Zentren, eines an der Verbindungsstelle des Stickstoffheterocyclischen Ringes mit dem Benzopyranteil (C-4 min), das andere beim durch R₄ substituierten Kohlenstoffatom (C-3 min), wodurch zwei optische Isomerenpaare möglich sind. Die Definition der erfindungsgemässen Verbindungen beeinhaltet alle möglichen Stereoisomere und deren Mischungen. Ganz besonders beeinhaltet sie die racemischen Formen und die isolierten optischen Isomeren mit der angegebenen Aktivität. Die zwei Racemate können durch physikalische Methoden, wie z.B. fraktionierte Kristallisation, getrennt werden. Die einzelnen optischen Isomere können von den Racematen durch Standardmethoden, wie z.B. Salzbildung mit einer optisch aktiven Säure und anschliessende Kristallisation, erhalten werden.

Geeignete Alkylgruppen für R₁ sind z.B. geradkettige oder verzweigte Radikale mit bis zu 6, vorzugsweise bis zu 5 Kohlenstoffatomen, z.B. Methyl-, Äthyl-, Propyl-, Isopropyl-, t-Butyl-, Pentyl- oder Isopentylgruppen.

Geeignete substituierte Alkylgruppen für R₁ sind z.B. Halogenalkyl, wie Trifluormethyl, Hydroxyalkyl, wie Hydroxyäthyl, oder Carboxyalkyl, wie Carboxyäthyl.

Geeignete Beispiele für eine Cycloalkylgruppe als R₁ mit 3 bis 6 Kohlenstoffatomen sind Cyclopropyl, Cyclobutyl, Cyclopentyl oder Cyclohexyl. Cyclopropylmethyl ist ein Beispiel für Cycloalkylalkyl.

Ein Beispiel für eine Arylalkylgruppe als R₁ ist eine Phenylalkylgruppe, in der die Phenylgruppe unsubstituiert oder einfach oder mehrfach durch Substituenten wie Halogen, C₁-C₄-Alkyl, C₁-C₄-Alkoxy, Nitro oder eine Trifluormethylgruppe, Aminogruppe oder eine substituierte Aminogruppe substituiert ist.

Ein Beispiel für eine Arylgruppe als R₁ ist eine Phenylgruppe, die unsubstituiert oder einfach oder mehrfach durch Substituenten wie Halogen, C₁-C₄-Alkyl, C₁-C₄-Alkoxy, Hydroxy, Carboxy, COO-Alkyl, CONH2. CONH-Alkyl, CON-(Alkyl)₂, Nitro oderTrifluormethyl, Amino, C₁-C₄-Alkylamino, Di-C₁-C₄-alkylamino, aromatische Heterocyclen wie Pyridylgruppen und polycyclische aromatische Reste wie Naphthylgruppen substituiert ist.

Ein geeignetes Beispiel für eine Alkylaminogruppe als R₁ ist (CH₂)ₙ-NR₆R₇, wobei n gleich 1 bis 3 ist und R₆ und R₇ Alkyl sind und dieselbe Bedeutung haben wie oben für Alkyl R₁ bis R₅ angegeben; ausserdem können R₆ und R₇ gemeinsam mit dem Stickstoffatom, an das sie gebunden sind, ein heterocyclischer Ring mit einem oder mehreren Heteroatomen sein. Geeignete Beispiele für heterocyclische Ringe, die von R₆ und R₇, gemeinsam mit dem Stickstoff, an den sie gebunden sind, gebildet werden, sind Piperidin, Pyrrolidin, Morpholin, Piperazin oder Imidazol, die unsubstituiert oder in einer oder mehreren Stellungen durch C₁-C₄-Alkyl, C₁-C₄-Alkoxy, Aryl oder eine Hydroxyl- oder Aminogruppe substituiert sein können.

Geeignete Beispiele für Salze der erfindungsgemässen Verbindungen mit anorganischen oder organischen Säuren sind das Hydrochlorid, Hydrobromid, Sulfat, Phosphat, Acetat, Oxalat, Tartrat, Citrat, Maleat oder Fumarat.

Ein wesentlicher und gravierender Nachteil der für die Behandlung von Tumorerkrankungen eingesetzten bekannten pharmazeutischen Zubereitungen liegt darin, daß derartige Zusammensetzungen teilweise erhebliche Nebenwirkungen beim Patienten hervorrufen, wobei die Art und Intensität dieser Nebenwirkungen abhängig vom jeweils vorhandenen Zytostatikum, dessen Konzentration und dessen chemischen Aufbau erheblich variieren können.

So ist es beispielsweise aus der EP 0 542 807 bekannt, die Nebenwirkungen des Zytostatikums Cisplatin durch die Anwesenheit oder gleichzeitige Gabe von 2-Phenyl-1,2-Benzisoselenazol-3(2H)-one (Ebselen) zu unterdrücken.

Der vorliegenden Erfindung liegt die Aufgabe zugrunde, eine pharmazeutische Zubereitung zur Behandlung von Tumorerkrankungen, insbesondere zur Behandlung von Krebs, zur Verfügung zu stellen, bei der besonders wirksam auch im längeren Gebrauch die vom zytostatischen Wirkstoff hervorgerufenen Nebenwirkungen reduziert und/ oder unterdrückt werden.

Diese Aufgabe wird erfindungsgemäß durch eine pharmazeutische Zubereitung mit den kennzeichnenden Merkmalen des Patentanspruchs 1 gelöst.

Erfindungsgemäß wird somit eine pharmazeutische Zubereitung zur Behandlung von Tumorerkrankungen, insbesondere zur Behandlung von Krebs, vorgeschlagen, wobei die erfindungsgemäße Zusammensetzung mindestens einen zytostatisch wirkenden Wirkstoff, insbesondere einen Wirkstoff auf der Basis von 4 H-1-Benzopyran-4-on, Taxan, Chinon, Benzochinon, Antrachinon sowie deren Derivate und/oder deren Salze, sowie üblichen Zusätze aufweist. Desweiteren ist in der erfindungsgemäßen Zubereitung wenigstens ein biologischer Elektronenakzeptor enthalten.

Die erfindungsgemäße pharmazeutische Zubereitung beruht auf der Grunderkenntnis, daß zytostatisch wirkende Wirkstoffe, die der Bekämpfung von Tumoren und insbesondere von Krebs dienen, energiereiche Elektronen aus der Mitochondrien-Membran aufnehmen und anschließend diese Elektronen an den, im Körper bzw. in den Zellen vorhandenen Sauerstoff weitergeben. Hierdurch werden toxische Sauerstoffradikale gebildet, die auch zusammengefaßt reaktive Oxygenspezies (ROSs) genannt werden. Diese energiereichen ROSs zerstören die chemische Struktur von Biomolekülen, aus denen der Organismus aufgebaut ist, so daß über diesen Weg entsprechende, teilweise sehr schwere Nebenwirkungen hervorgerufen werden.

Überraschend wurde nunmehr gefunden, daß die zuvor angesprochenen überschüssigen und/oder fehlgeleiteten energiereichen Elektronen aus der Mitochondrien-Membran von tierischen und/oder menschlichen Zellen abgefangen werden können, bevor diese Elektronen mit Sauerstoff unter Bildung der toxischen ROSs reagieren können. Hierfür ist es erforderlich, daß die erfindungsgemäße pharmazeutische Zubereitung entsprechende biologische Elektronenakzeptoren aufweist, wobei der wenigstens eine, in der erfindungsgemäße Zubereitung enthaltene biologische Elektronenakzeptor die überschüssigen und/oder fehlgeleiteten energiereichen Elektronen und somit die Bildung von ROSs als toxischer Metabolit verhindert. Hienn wird die Ursache gesehen, warum die erfindungsgemäße pharmazeutische Zubereitung eine deutliche Verringerung der Nebenwirkungen und somit eine erhebliche Verbesserung der Verträglichkeit der zytostatischen Wirkstoffe herbeiführt, so daß bei Anwendung der erfindungsgemäßen Zubereitung zur Tumor- und insbesondere zur Krebsbehandlung, unerwünschte Nebenwirkungen vollstandig oder nahezu vollständig unterdrückt werden, selbst dann, wenn die erfindungsgemäße pharmazeutische Zubereitung über einen langen Zeitraum dargereichtwird. Desweiteren können die in der erfindungsgemäßen pharmazeutischen Zubereitung enthaltenen biologischen Elektronenakzeptoren die Löslichkeit gewisser zytostatisch wirkender Wirkstoffe verbessern oder die Resorbierbarkeit dieser Wirkstoffe erhöhen, so daß hierdurch zusätzliche Vorteile zur Verfügung gestellt werden, wie dies nachfolgend noch im Detail erläutert ist.

Mit anderen Worten enthält somit die erfindungsgemäße pharmazeutische Zubereitung wenigstens einen biologischen Elektronenakzeptor, wobei dieser biologische Elektronenakzeptor dadurch definiert wird, daß er in der Lage ist, im menschlichen und/oder tierischen Körper die bei der Gabe eines zytostatischen Wirkstoffes entstehenden oder auftretenden energiereichen Elektronen abzufangen, so daß die Bildung von ROSs wirksam unterdrückt wird. Der biologische Elektronenakzeptor selbst ist untoxisch.

Der biologische Elektronenakzeptor enthält eine funktionelle Gruppe der allgemeinen Formel 1

- (CH₂)₂ - N⁺ - (CH₃)₃ (Formel 1)

Zu dem biologischen Elektronenakzeptor, der mindestens eine funktionelle Gruppe der vorstehend wiedergegebenen allgemeinen Formel 1 enthält, wurde überraschend gefunden, daß unerwünschte energiereiche Elektronen, die, wie vorstehend bereits erklärt, fehlgeleitet oder im Überschuß vorhanden sind, durch die zuvor wiedergegebene und in Formel 1 dargestellte funktionelle Gruppe abgefangen werden.

Im Rahmen dieser Abfangreaktion werden zwei Elektronen und ein Proton in Form von einem Hydride-Ion - H- - auf die funktionelle Gruppe des Elektronenakzeptors unter Abspaltung von Methan übertragen. Dies führt dazu, daß das Hydride-Ion und somit die energiereichen Elektronen in den Membranen der Mitochondrien nicht an Sauerstoff übertragen werden sondern in Form von Methan den Organismus verlassen können.

Daß die zuvor beschriebene Abfangreaktion auch tatsächlich so abläuft, wenn biologische Organismen mit der erfindungsgemäßen pharmazeutischen Zubereitung behandelt werden, konnte anhand von Versuchen mit Leberzellen sowie in Tierversuchen nachgewiesen werden, wobei als Folge von derartigen überschüssigen, jedoch bei der erfindungsgemäßen Zubereitung abgefangenen energiereichen Elektronen die Entstehung von Methan nachgewiesen wurde. Mit dieser Abfangreaktion konnte die toxische, überschüssige Energie in ein für den menschlichen oder tierischen Organismus indifferentes, nicht-toxisches Gas umgewandelt werden, das durch die Lunge ausgeatmet wird.

Insbesondere weist die erfindungsgemäße pharmazeutische Zubereitung als biologischen Elektronenakzeptor S-Adenosylmethionin, ein Derivat und/oder ein Salz hiervon auf. Das bei dieser Ausführungsform der erfindungsgemäßen Zubereitung hierin als biologischer Elektronenakzeptor enthaltene S-Adenosylmethionin, dessen Derivat und/oder dessen Salz fungiert ebenfalls mittels einer darin enthaltenen mindestens einen Methylgruppe als Elektronenakzeptor und fangt somit überschüssige, bei der Anwendung der zytostatischen Wirkstoffe entstehende energiereiche und/oder fehlgeleitete Elektronen ab, so daß dementsprechend mindestens eine Methylgruppe abgespalten und in unschädliches Methan umgewandelt wird, so daß diese Elektronen nicht zu einer Beeinträchtigung und/oder Schädigung von Zellen führen können, die letztendlich eine Hauptursache für die bei der Gabe von Zytostatika auftretenden Nebenwirkungen sind.

Die erfindungsgemäße pharmazeutischen Zubereitung weist einen biologischen Elektronenakzeptor auf, bei dem es sich um einen natürlichen, in aeroben Zellen vorhandenen Elektronenakzeptor handelt, nämlich Verbindungen der nachstehenden Formel A, die aus biologischem Material, so vorzugsweise Soja-Bohnen, Mais, Weizen, Raps, Ölfrüchten und Ölsaaten und/oder Eiern, isoliert oder die synthetisch oder halbsynthetisch hergestellt bzw. derivatisiert werden.

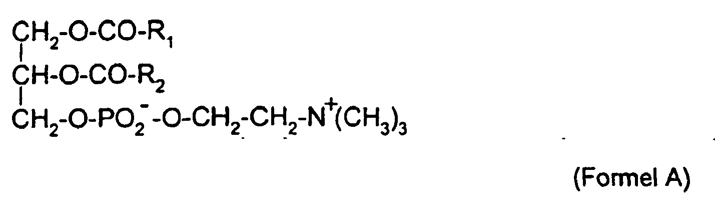

In Formel A können R₁ und R₂ identisch oder verschieden sein und jeweils für Wasserstoff und/oder für den Rest einer gesättigten und/oder ungesättigten C₁-C₂₂-Fettsäure, vorzugsweise für Palmitin-, Stearin-, Öl-, Linol- und/ oder Linolensäure, stehen.

Bei den synthetischen bzw. halbsynthetischen Verbindungen bzw. Derivaten sind vorzugsweise Dipalmitoylphosphatidylcholin (DPPC), Distearylphosphatidylcholin (DSPC) und Dimyristoylphosphatidylcholin (DMPC) zu nennen.

Auch durch solche Ausgestaltungen der erfindungsgemäßen pharmazeutischen Zubereitung, bei denen die Zubereitung als biologischen Elektronenakzeptor, Glycerophosphocholin, Phosphatidylcholin, Lysophosphatidylcholin, sowohl als Einzelsubstanzen als auch als Mischungen und/oder Derivate enthält, wird die unerwünschte Ausbildung der ROSs und damit das Auftreten von Nebenwirkungen deutlich unterdrückt.

Besonders geeignete und eine hohe Unterdrückungskapazität bezüglich der Nebenwirkungen aufweisende Ausgestaltungen der erfindungsgemäßen pharmazeutischen Zubereitung enthalten den biologischen Elektronenakzeptor und den zytostatisch wirkenden Wirkstoff in einem molaren Massenverhältnis zwischen 0,1:1 und 5:1, vorzugsweise in einem molaren Massenverhältnis zwischen 0,5:1 und 2:1.

Eine besonders geeignete und vorteilhaft anzuwendende sowie lagerstabile Ausgestaltung der erfindungsgemäßen pharmazeutischen Zubereitung sieht vor, daß hierbei die erfindungsgemäße Zubereitung als biologischen Elektronenakzeptor ein Phospholipid, insbesondere ein pflanzliches Phospholipid und vorzugsweise ein Soja-Phospholipid, aufweist. Hierbei bewirken diese phospholipidischen biologischen Elektronenakzeptoren, daß einerseits sehr wirksam die Nebenwirkungen unterdrückt werden, und andererseits es ermöglicht wird, daß solche zytostatischen Wirkstoffe, die schlecht löslich sind, erheblich besser in einem geeigneten nicht toxischen Lösungsmittel gelöst bzw. stabil dispergiert bzw. stabil emulgiert werden können. Darüber hinaus ist es über die Verwendung derartiger Phospholipide als biologische Elektronenakzeptoren möglich, Emulsionen, Nanoemulsionen, liposomale Formulierungen, mischmizellenhaltige Formulierungen oder sogar die Ausbildung von Komplexen zwischen den Phospholipiden und den zytostatisch wirkenden Wirkstoffen zu erstellen, so daß dementsprechend derartige Formulierungen, die den phospholipidischen biologischen Elektronenakzeptor sowie den mindestens einen zytostatisch wirkenden Wirkstoff aufweisen, eine Reihe von weiteren Vorteilen besitzen. Diese drücken sich beispielsweise darin aus, daß der zytostatisch wirkende Wirkstoff besser und/oder leichter lösbar, dispergierbar bzw. emulgierbar ist, wodurch beispielsweise die Dosierung von flüssigen Zubereitungen erleichtert wird, daß die Lagerstabilität erhöht wird, daß die Sterilfiltrierbarkeit gegeben ist, daß eine Transparenz gewährleistet wird oder daß zusätzlich der Wirkstoff in einem entsprechenden phospholipidischen Vesikel eingekapselt wird, wodurch eine größere Konzentration an Wirkstoff schneller und mit einem höheren Wirkungsgrad verabreicht werden kann.

Die zuvor genannten Vorteile treten insbesondere dann auf, wenn die erfindungsgemäße pharmazeutische Zubereitung als zytostatisch wirkenden Wirkstoff 4H-1-Benzopyran-4-on und/oder ein Derivat oder Salz davon enthält, so insbesondere das im Europäischen Patent 0 366 061 als bevorzugte Verbindung beschriebene Flavopiridol-HCl, wobei Flavopiridol durch die Formel C beschrieben wird:

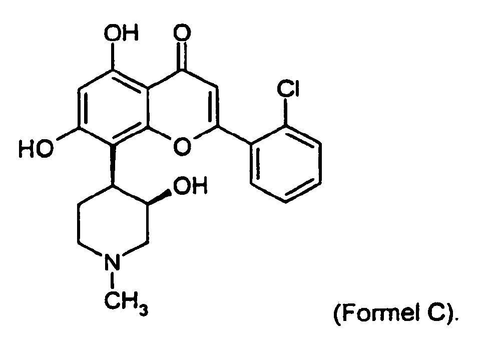

Überraschend konnte festgestellt werden, daß solche Zubereitungen, die als biologischen Elektronenakzeptor Phospholipide, insbesondere die zuvor genannten oder die nachfolgend noch beschriebenen speziellen Phospholipide, enthalten, eine hervorragende Lagerstabilität unter Beibehaltung der zytostatischen Wirksamkeit besitzen, obwohl zu befürchten war, daß insbesondere bei solchen Ausführungsformen, die Phosphatidylcholin als biologischen Elektronenakzeptor und einen Wirkstoff auf der Basis von 4H-1-Benzopyran-4-on, dessen Derivaten und/oder Salzen und vorzugsweise Flavopiridol enthalten, während der Lagerung eine unerwünschte Wechselwirkung zwischen dem biologischen Elektronenakzeptor und dem Wirkstoff zeigen, die zu einer Desaktivierung und/oder zu einer unerwünschten Modifizierung des Wirkstoffes und/oder des Elektronenakzeptors geführt hätte. Besonders geeignete Ausgestaltungen der erfindungsgemäßen pharmazeutischen Zubereitung weisen solche phospholipidischen biologischen Elektronenakzeptoren auf, bei denen das zugrundeliegende Phospholipid, insbesondere das aus Soja-Bohnen isolierte Phospholipid, eine Konzentration an Phosphatidylcholin von wenigstens 50 Gew.%, bezogen auf die Gesamtmenge des in der Zubereitung enthaltenen Elektronenakzeptors, enthält. Hier sind beispielsweise als besonders geeignet solche phospholipidischen biologischen Elektronenakzeptoren zu nennen, die neben mindestens 50 Gew.% Phosphatidylcholin ein flüssiges Trägersystem, insbesondere einen pharmazeutisch akzeptablen, primären C₂-C₄-Alkohol und/ oder ein natürliches Öl und/oder ein Polyalkylenglykol, enthalten. Bezüglich der öligen Komponente in derartigen flüssigen Phosphatidylcholin-Zubereitungen ist festzuhalten, daß hierfür insbesondere flüssige Triglyceride bevorzugt sind, so beispielsweise Caprylsäure-/Caprinsäure-Triglyceride, Glycerinstearate, Ascorbinpalmitate, Oleylsäurepalmitate, Kokosnußöl, Polyethylenglykol und/oder Polyethylenglykol, jeweils allein oder in Mischung, wobei die Konzentration an Phosphatidylcholin in derartigen öligen Zubereitungen dann vorzugsweise zwischen 45 Gew.% und 75 Gew. %, insbesondere zwischen 50 Gew.% und 60 Gew.%, variiert.

Ist hingegen bei der erfindungsgemäßen pharmazeutischen Zubereitung eine höhere Menge an energiereichen Elektronen und/odertoxischen Sauerstoffradikalen (ROSs) aufzufangen, so gelangen als phospholipidische biologische Elektronenakzeptoren solche phospholipidischen Zusammensetzungen zur Anwendung, deren Konzentration an Phosphatidylcholins größer als 70 Gew.% und vorzugsweise größer als 80 Gew.% und insbesondere größer als 90 Gew.%, ist, wobei sich diese Konzentrationsangabe auf die Gesamtmenge des in der Zubereitung enthaltenen phospholipidischen Elektronenakzeptors beziehen. So kann insbesondere die erfindungsgemäße Zubereitung einen solchen phospholipidischen biologischen Elektronenakzeptor enthalten, der flüssig formuliert ist und der zwischen 70 und 80 Gew.% Phosphatidylcholin neben den zuvor aufgeführten öligen Substanzen aufweist. Hochreine phospholipidische biologische Elektronenakzeptoren weisen dann zwischen 90 Gew.% und 96 Gew.% Phosphatidylcholin, bezogen auf die Menge des phospholipidischen biologischen Elektronenakzeptors, auf.

Eine weitere, besonders vorteilhafte Ausgestaltung der erfindungsgemäßen pharmazeutischen Zubereitung sieht vor, daß der zuvor wiedergegebene phospholipidische biologische Elektronenakzeptor neben der vorstehend genannten Konzentration an Phosphatidylcholin mindestens noch ein negativ geladenes Phospholipid, insbesondere N-Acylphosphatidylethanolamin, Phosphatidylinositot, Phosphatidylglycerol, Phosphatidsäure sowie Salze und/oder Derivate der zuvor genannten negativ geladenen Phospholipide enthält. Hierbei bewirken diese negativ geladenen Phospholipide, daß entsprechende flüssige Formulierungen der erfindungsgemäßen pharmazeutischen Zubereitung eine hohe Transparenz aufweisen, selbst wenn der zytostatisch wirkende Wirkstoff in dem jeweils verwendeten Lösungsmittel nicht löslich ist, und daß die Lagerstabilität entsprechend erhöht ist, so daß selbst bei einer extrem langen Lagerzeit kein Bodensatz ausgebildet wird.

Vorzugsweise variiert die Konzentration der negativ geladenen Phospholipide bei der zuvor beschriebenen Ausführungsform der erfindungsgemäßen Zubereitung zwischen 2 Gew.% und 10 Gew.%, bezogen auf die Gesamtmenge des in der Zubereitung enthaltenen phospholipidischen biologischen Elektronenakzeptors.

Vorstehend ist im Zusammenhang mit dem phospholipidischen biologischen Elektronenakzeptor beschrieben worden, daß dieser ein Phospholipid bzw. ein Phospholipidgemisch, insbesondere auch Phosphatidylcholin, umfaßt. Hierunter fallen neben dem bereits mehrfach genannten 1,2-Diacylglycero-3-Phosphocholin[(3-sn-Phosphatidyl)cholin] auch vorzugsweise 1,2-Diacylglycero-3-Phosphoethanolamin, 1,2-Diacylglycero-3-Phosphoinositol, 1,2-Diacylglycero-3-Phosphoserin, 1,2-Diacylglycero-3-Phosphoglycerol und 1,2-Diacylglycero-3-Phosphatjeweils allein oder in Mischung.

Bei einer besonders geeigneten Weiterbildung der zuvor beschriebenen erfindungsgemäßen Zubereitungen enthält diese ein solches Phosphatidylcholin, bei dem die im Phosphatidylcholin enthaltenen Acylreste zu
61 - 73 Gew.% aus dem Linolsäurerest,
10 - 14 Gew.% aus dem Palmitinsäurerest,
8 - 12 Gew.% aus dem Ölsäurerest,
4 - 6 Gew.% aus dem Linolensäurerest,
3 - 5 Gew.% aus dem Stearinsäurerest und
bis zu 2 Gew.% aus anderen Fettsäureresten
bestehen.

Wie bereits vorstehend erwähnt, kann das in der erfindungsgemäßen Zubereitung vorgesehene Phospholipid ein Phospholipidgemisch sein. Hierfür kommen insbesondere die bereits vorstehend genannten 1,2-Diacylglycero-3-phosphat (1,2-Diacylglycero-3-phosphoethanolamin, 1,2-Diacylglycero-3-phosphoinositol, 1,2-Diacylglycero-3-phosphoserin, 1,2-Diacylglycero-3-phosphoglycerol und/oder 1,2-Diacylglycero-3-phosphat) in Frage, wobei vorzugsweise bis zu 30 Gew.% der vorstehend genannten 1,2-Diacylglycero-3-phosphate in dem Phospholipidgemisch enthalten sind, während eine derartige Ausführungsform der erfindungsgemäßen Zubereitung dann mindestens 70 Gew.% des 1,2-Diacylglycero-3-phosphocholin aufweist. Hierbei beziehen sich die zuvor genannten prozentualen Massenangaben auf die Gesamtmasse des in der erfindungsgemäßen Zubereitung enthaltenen phospholipidischen biologischen Elektronenakzeptors.

Eine andere, ebenfalls besonders geeignete Ausführungsform der erfindungsgemäßen Zubereitung beinhaltet als Phospholipid ein 1,2-Diacylglycero-3-phosphocholin, bei dem der 1-Acylrest
45 - 61 Gew.% Linolsäurereste,
19 - 26 Gew.% Palmitinsäurereste,
8 - 12 Gew.% Ölsäurereste,
4 - 6 Gew.% Linolensäurereste,
6 - 9 Gew.% Stearinsäurereste und
2 Gew.% andere Fettsäurereste
umfaßt, während der 2-Acylrest zu
77 - 85 Gew.% aus dem Linolsäurerest,
1 - 2 Gew.% aus dem Palmitinsäurerest,
8 - 12 Gew.% aus dem Ölsäurerest,
4 - 6 Gew.% aus dem Linolensäurerest,
0 - 1 Gew.% aus dem Stearinsäurerest und
2 Gew.% aus anderen Fettsäureresten
besteht.

Bezüglich der jeweiligen Formulierung der erfindungsgemäßen pharmazeutischen Zubereitung ist festzuhalten, daß hierbei jede Formulierung, die eine orale, parenterale und/odertopische Darreichung der erfindungsgemäßen Zubereitung erlaubt, geeignet ist. Dementsprechend wird die erfindungsgemäße Zubereitung als Tablette, Kapsel, Lösung, Emulsion, Dispersion, Liposomensystem und/oder als flüssiges Mischmizellensystem aufbereitet.

Auch dragierte Formulierungen und dragierte Retardformulierungen gehören in den Rahmen der Erfindung. Bevorzugt sind säure- und magensaftresistente Formulierungen. Pharmazeutisch übliche Zusätze umfassen magensaftresistente Beschichtungen wie Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, als Pulver oder Granulate; als Lösung oder Suspension in einer wäßrigen oder nicht-wäßrigen Flüssigkeit; oder als eine Öl-in-Wasser- oder Wasserin-Öl-Emulsion. Diese Zusammensetzungen können nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem pharmazeutisch üblichen Zusatz wie einem Bindemittel, einem Gleitmittel, einem inerten Verdünner und/oder einem oder mehreren oberflächenaktiven/ dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die als pharmazeutisch verträgliche Zusätze üblicherweise Saccharose und Gummi arabicum oder Tragant enthalten, und Pastillen, die die Verbindung in einer inerten Basis wie Gelatine und Glycerin oder Saccharose und Gummi arabicum umfassen.

Orale und perorale Zubereitungen können gegebenenfalls weitere pharmazeutisch übliche Zusätze enthalten, beispielsweise einen Aromastoff, insbesondere ein Fruchtaroma, oder Süßstoffe, beispielsweise Saccharin-Natrium.

Geeignete pharmazeutische Zusammensetzungen für die parenterale Verabreichung umfassen vorzugsweise sterile wäßrige Zubereitungen, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird.

Geeignete pharmazeutische Zusammensetzungen für die topische Anwendung auf der Haut liegen vorzugsweise als Salbe, Creme, Lotion, Paste, Spray, Aerosol oder Öl vor. Als pharmazeutisch übliche Zusätze können Träger wie beispielsweise Vaseline, Lanolin, Polyethylenglycole, Alkohole und Kombinationen von zwei oder mehreren dieser Substanzen verwendet werden.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise Wirkstoff und Elektronenakzeptor in einer gegebenenfalls gepufferten wäßrigen Lösung, gelöst und/oder dispergiert in einem Haftmittel oder dispergiert in einem Polymer. Als eine besondere Möglichkeit kann der Wirkstoff, wie beispielsweise in Pharmaceutical Research, 2(6): 318 (1986) beschrieben, durch Elektrotransport oder lonophorese freigesetzt werden.

Eine besonders geeignete und relativ einfach zu dosierende Ausgestaltung der erfindungsgemäßen Zubereitung sieht vor, daß hierbei die Zubereitung als Injektions- oder Infusionsflüssigkeit formuliert ist, wobei diese Zubereitung dann vorzugsweise als zytostatisch wirkenden Wirkstoff Flavopiridol-HCl, Doxorubicin-HCl, Idarubicin-HCl und/ oder Daunorubicin-HCl aufweist. Die zuvor genannten Wirkstoffe sind dann in einem geeigneten Lösungsmittel als pharmazeutisch üblichem Zusatz, so beispielsweise Wasser, Ethanol, Propanol, Isopropanol und/oder Mischungen davon gelöst, dispergiert, emulgiert und/oder in Form von Liposomen und/oder Mischmizellen aufbereitet. Desweiteren können in diesen flüssigen Zubereitungen die zuvor genannten Öle und/oder Polyalkylenoxide vorhanden sein. Ferner weisen diese flüssigen Darreichungsformen als biologischen Elektronenakzeptor, Phospholipide, vorzugsweise die zuvor aufgeführten konkreten Phospholipide und insbesondere Phosphatidylcholin, auf.

Bezüglich der Konzentration des zytostatischen Wirkstoffes in den zuvor beschriebenen flüssigen Darreichungsformen ist festzuhalten, daß sich diese Konzentration nach dem jeweilig ausgewähltem Lösungsmittel sowie der Löslichkeit, der Dispergierbarkeit bzw. der Emulgierbarkeit des jeweiligen zytostatischen Wirkstoffes in diesem Lösungsmittel richtet. Als besonders geeignet haben sich hierbei Konzentration des zytostatischen Wirkstoffes erwiesen, die zwischen 1 mg und 200 mg, insbesondere zwischen 5 mg und 40 mg, variieren.

Abhängig von der Konzentration des zytostatischen Wirkstoffes und dem chemischen Aufbau desselben wird in der erfindungsgemäßen pharmazeutischen Zubereitung auch die Konzentrationen an biologischem Elektronenakzeptor festgelegt. Hier hat sich gezeigt, daß vorzugsweise die erfindungsgemäße Zubereitung bei einer flüssigen Formulierung Konzentrationen an biologischem Elektronenakzeptor zwischen 50 mg und 3 mg, insbesondere zwischen 250 mg und 1 mg, aufweist, wobei auch die Art des jeweils verwendeten biologischen Elektronenakzeptors, d.h. sein chemischer Aufbau, einen Einfluß auf die Konzentration des jeweils einzusetzenden biologischen Elektronenakzeptors hat.

Wird hingegen die erfindungsgemäße pharmazeutische Zubereitung als oral darzureichende Zubereitung, so insbesondere als Tablette, Granulat oder Pulver, formuliert, so weist vorzugsweise die erfindungsgemäße Zubereitung als zytostatisch wirkenden Wirkstoff Flavopiridol-HCl und/oder Idarubicin-HCl auf, während als biologischer Elektronenakzeptor Betaindihydrogencitrat, Cholincitrat, Phospholipide und insbesondere Phosphatidylcholin und/oder Ademethionintosylatbis(sulfat) vorgesehen ist.

Wie bereits bei den zuvor beschriebenen flüssigen Zubereitungen dargelegt wurde, richtet sich die Konzentration des zytostatischen Wirkstoffes in den oral darzureichenden Formulierungen nach dem jeweils ausgewählten Wirkstoff bzw. Wirkstoffgemisch, wobei bevorzugte Konzentrationen des zytostatischen Wirkstoffes zwischen 5 mg und 70 mg, insbesondere zwischen 15 mg und 40 mg, variieren.

Insbesondere weisen die zuvor beschriebenen oralen Darreichungsformen Konzentrationen an biologischem Elektronenakzeptor auf, die zwischen 50 mg und 3 mg, vorzugsweise zwischen 250 mg und 1 mg, liegen.

Zuvor wurden zu der erfindungsgemäßen pharmazeutischen Zubereitung Ausführungsformen beschrieben, bei denen die erfindungsgemäße Zubereitung gleichzeitig mindestens einen zytostatisch wirkenden Wirkstoff und zusätzlich noch den biologischen Elektronenakzeptor enthält.

Eine andere, besonders geeignete Ausführungsvariante sieht vor, daß hierbei die erfindungsgemäßen pharmazeutische Zubereitung zur Behandlung von Tumorerkrankungen, insbesondere zur Behandlung von Krebs, zwei flüssige oder zwei feste Zubereitungen oder eine feste und eine flüssige Zubereitung umfaßt, wobei eine erste Zubereitung mindestens einen zytostatisch wirkenden Wirkstoff auf der Basis von 4H-1-Benzopyran-4-one, Taxan, Chinon, Benzochinon, Anthrachinon, deren Derivate und/oder deren Salze, sowie übliche Zusätze aufweist, während eine zweite Zubereitung dann den biologischen Elektronenakzeptor enthält. Mit anderen Worten ist bei dieser Ausgestaltung der erfindungsgemäßen pharmazeutischen Zubereitung die erste Zubereitung getrennt von der zweiten Zubereitung formuliert, so daß insbesondere dann, wenn diese beiden Zubereitungen flüssig oder pulverförmig dargereicht werden, eine individuelle Dosis des zytostatischen Wirkstoffes gegeben werden kann und zusätzlich vorher, gleichzeitig oder danach eine von der Reaktion des zu behandelnden Patienten abhängig gewählte und individuell hierauf angepaßte Konzentration des biologischen Elektronenakzeptors dem Patienten zur Verfügung gestellt wird. Bei dieser Ausgestaltung der erfindungsgemäßen Zubereitung gelten sinngemäß die Ausführungen, wie sie zuvor für die Ausführungsformen dererfindungsgemäßen Zubereitung beschrieben sind, die in einer Formulierung Wirkstoff und biologischen Elektronenakzeptor enthalten.

Die vorliegende Erfindung betrifft insbesondere auch die Verwendung der zuvor beschriebenen pharmazeutischen Zubereitung zur Behandlung von Tumoren, insbesondere zur Behandlung von Krebs, wobei die erfindungsgemäße Zubereitung in einer täglichen Dosis von 0,0001 g und 2 g, insbesondere zwischen 0,01 g und 1 g, des zytostatischen Wirkstoffes und in einer täglichen Dosis zwischen 0,1 g und 100 g, insbesondere zwischen 5 g und 50 g, des biologischen Elektronenakzeptors dargereicht wird, wobei sich die zuvor genannten Dosisraten jeweils auf einen Quadratmeter der Körperoberfläche des zu behandelnden Patienten beziehen.

Die erfindungsgemäße Zubereitung wird nachfolgend anhand von Ausführungsbeispielen näher erläutert ohne diese jedoch einzuschränken.

### Ausführungs- und Vergleichsbeispiele A1 bis A5

Die nachfolgenden Ausführungsbeispiele A1, A2 und A5 betreffen solche Zubereitungen, die für die parenterale Anwendung bestimmt sind und die unterschiedliche zytostatisch wirkende Wirkstoffe sowie unterschiedliche biologische Elektronenakzeptoren enthalten.

Zur Herstellung der Zubereitungen A1, A2 und A5 wurden die in Tabelle 1 genannten Bestandteile in den dort genannten Mengen jeweils in Ethanol gelöst. Nach Abzug des Lösungsmittels unter Vakuum und Inertgas wurde der verbliebene Rückstand in 20 I (Zubereitung A1 und A5) bzw. 10 I (Zubereitung A2) Wasser dispergiert. Anschließend erfolgte eine Homogenisierung der Dispersion unter Ausbildung von Liposomen mit einem mittleren Partikeldurchmesser zwischen 0,1 µm und 1 µm.

Zu den homogenisierten Dispersionen wurden zu den Zubereitungen A1 und A5 jeweils 2 kg Maltose, gelöst in 2 I Wasser, und bei der Zubereitung A2 1 kg Maltose, gelöst in 1 I Wasser, zugesetzt, wobei die diesbezügliche Dispersion nochmals homogenisiert wurde.

Hierbei resultierten liposomale Formulierungen mit einem mittleren Liposomendurchmesser zwischen 0,1µm und 1 µm.

Die homogenen Mischungen wurden unter Verwendung eines Filters mit einer Porengröße von 0,2 µm steril filtriert.

Die so steril filtrierten Zubereitungen wurden in Vials abgefüllt, wobei jedes Vial der Zubereitung A1 100 mg Flavopiridol in 20 ml, die Zubereitung A2 10 mg Doxorubicin in 20 ml und die Zubereitung A5 100 mg Flavopiridol in 20 ml enthielten.

Zur Lagerung wurden die diesbezüglichen befüllten Vials anschließend gefriergetrocknet.

Zur Infusion wird dann jedes Vial mit 20 ml Wasser re-dispergiert und nach Zusatz von 250 ml Glukoselösung (Glukosekonzentration: 5 Gew.%) gemischt.

**Tabelle 1**

| | A1 | A2 | A5 |
|---|---|---|---|
| Flavopiridol-HCl | 100 g | - | 100 g |
| Doxorubicin | - | 10 g | - |
| Phosphatidylcholin | 2000 g | 1000 g | 2000 g |
| DSPG | 40 g | 20 g | 40 g |
| Betainlinolat | - | - | 250 g |
| Ethanol | 10 l | 5 l | 10 l |
| DSPG = Disteroylphosphatidylglycerol | | | |
| Phosphatidylcholin = Phospholipon 90; Phospholipidkonzentration: 93 + 3 Gew.% | | | |

Zur Herstellung der Zubereitungen A3 und A4 wurden die in der Tabelle 2 genannten Bestandteilen in den dort genannten Mengen jeweils in Wasser gelöst. Hiernach wurden die Lösungen steril filtriert unter Verwendung eines Filters mit einer Porengröße von 0,2 µm. Nach Abfüllung der steril filtrierten Lösung mit einer Konzentration von 10 mg des zytostatisch wirkenden Wirkstoffes in 10 ml Vials wurden die Vials gefriergetrocknet.

Unmittelbar vor der Infusion wurden die so gefriergetrockneten Inhaltsstoffe mit 10 ml Wasser re-dispergiert und mit 200 ml Glukoselösung (Glukosekonzentration: 5 Gew.%) gemischt.

**Tabelle 2 (Vergleichsbeispiele)**

| | A3 | A4 |
|---|---|---|
| Doxorubicin | - | 10 g |
| Idarubicin | 10 g | - |
| Betaindihydrogencitrat | 200 g | - |
| Cholincitrat | - | 250 g |
| Lactose | 200 g | 250 g |
| Wasser | 10 l | 10 l |

Ausführungs- und Vergleichsbeispiele B1 bis B5

Die nachfolgenden Vergleichsbeispiele B1 B2, B4, B5 und Ausführungsbeispiel B3 betreffen solche Zubereitungen, die für die parenterale Anwendung bestimmt sind und bei denen die unterschiedlichen zytostatisch wirkenden Wirkstoffe sowie die unterschiedlichen biologischen Elektronenakzeptoren getrennt voneinander hergestellt und gelagert werden, so daß erst unmittelbar vor der parenteralen Anwendung die den zytostatisch wirkenden Wirkstoff enthaltende erste Zubereitung mit der den biologischen Elektronenakzeptor enthaltenden zweite Zubereitung vermischt wird.

Zur Herstellung der ersten Zubereitung, die den zytostatisch wirkenden Wirkstoff enthält, wurden die in der Tabelle 3 genannten Bestandteile in den dort genannten Mengen gelöst. Hiernach wurde die Lösung unter Verwendung eines 0,2 µm Filters steril filtriert, wobei bei der Zubereitung B11 mg Fluorouracil in 40 ml Ampullen, bei der Zubereitung B2 20 mg Daunorubicin in 20 ml Vials, bei der Zubereitung B3 10 mg Doxorubicin in 5 ml Vials, bei der Zubereitung B4 10 mg Idarubicin in 5 ml Vials und bei der Zubereitung B5 10 mg Mitomycin in 10 ml Vials abgefüllt wurden.

Die mit den Zubereitungen B2 bis B5 gefüllten Vials wurden gefriergetrocknet und entsprechend gelagert.

Zur Herstellung der zweiten Zubereitungen B1, B2, B4 und B5, die den biologischen Elektronenakzeptor enthalten, wurden die in der Tabelle 4 genannten Bestandteile in den dort aufgeführten Mengen in Wasser gelöst. Hiernach wurde die diesbezügliche Lösung unter Verwendung eines 0,2 µm Filters steril filtriert.

Bei der zweiten Zubereitung B1 wurden 500 mg Betaindihydrogencitrat in 10 ml Ampullen, bei der Zubereitung B2 100 mg Cholincitrat in 5 ml Ampullen, bei der Zubereitung B4 200 mg Cholincitrat in 10 ml Ampullen und bei der Zubereitung B5 250 mg Betaindihydrogencitrat in 5 ml Ampullen abgefüllt.

Die Herstellung der zweiten Zubereitung 83 erfolgte derart, daß 10 kg Phosphatidylcholin zusammen mit 20 g DSPG (Distearoylphosphatidylglycerol) in 20 l Ethanol gelöst wurden. Hiernach wurde das Ethanol unter Vakuum und Inertgas abgezogen. Der Rückstand wurde mit 20 l Wasser dispergiert und anschließend unter Ausbildung einer liposomalen Formulierung homogenisiert, wobei der Liposomendurchmesser zwischen 0,1 µm und 1 µm variierte.

Zu dieser liposomalen Formulierung wurde dann eine Lösung, bestehend aus 10 kg Maltose und 10 l Wasser, zugesetzt. Anschließend erfolgte eine Vermischung so lange, bis eine transparente, homogene Dispersion entstand.

Die so hergestellte Dispersion wurde unter Verwendung eines 0,2 µm Filters steril filtriert.

Die steril filtrierte Dispersion wurde mit 1 g Phosphatidylcholin in 20 ml Vials abgefüllt. Hiernach erfolgte eine Gefriertrocknung der Vials.

**Tabelle 3**

| Zusammensetzung der ersten, den zytostatisch wirkenden Wirkstoff enthaltenden Zubereitungen B1 bis B5 | | | | | |
|---|---|---|---|---|---|
| | B1 | B2 | B3 | B4 | B5 |
| Fluorouracil | 1 g | - | - | - | - |
| Daunorubicin | - | 100 g | - | - | - |
| Doxorubicin | - | - | 100 g | - | - |
| Idarubicin | - | - | - | 100 g | - |
| Mitomycin | - | - | - | - | 10 g |
| Maltose | - | 200 g | - | - | - |
| Lactose | - | - | 10 kg | 10 kg | 100 kg |
| Wasser | 10 l | 20 l | 50 l | 50 l | 10 l |

**Tabelle 4**

| Zusammensetzung der zweiten, den biologischen Elektronenakzeptor enthaltenden Zubereitungen B1, B2, B4 und B5 (Vergleichsbeispiele) | | | | |
|---|---|---|---|---|
| | B1 | B2 | B4 | B5 |
| Betaindihydrogencitrat | 500 g | - | - | 250 g |
| Cholincitrat | - | 100 g | 200 g | - |
| Wasser | 10 l | 5 l | 10 l | 5 l |

Unmittelbar vor der Infusion wurde eine Ampulle der ersten Zubereitung B1 mit einer Ampulle der zweiten Zubereitung B1 unter Zusatz von 250 ml Glukoselösung (Glukosekonzentration: 5 Gew.%) vermischt.

Unmittelbar vor der Infusion wurde ein Vial der ersten Zubereitung B2 in 20 ml Wasser re-dispergiert und mit einer Ampulle der zweiten Zubereitung B2 unter Zusatz von 250 ml Glukoselösung (Glukosekonzentration: 5 Gew.%) vermischt.

Unmittelbar vor der Infusion wurde ein Vial der ersten Zubereitung B3 mit 5 ml Wasser re-dispergiert und mit einem Vial der zweiten Zubereitung B3 versetzt, wobei zuvor die zweite Zubereitung mit 20 ml Wasser re-dispergiert worden ist. Hierzu erfolgte noch ein Zusatz und ein Vermischen mit 250 ml Glukoselösung (Glukosekonzentration: 5 Gew.%).

Unmittelbar vor der Infusion wurde ein Vial der ersten Zubereitung B4 mit 10 ml Wasser re-dispergiert. Hierzu erfolgte ein Zusatz einer Ampulle der zweiten Zubereitung B4, wobei zuvor noch ein Zusatz und ein Vermischen mit 200 ml Glukoselösung (Glukosekonzentration: 5 Gew.%) durchgeführt worden ist.

Die erste Zubereitung B5 wurde unmittelbar vor der Infusion mit 10 ml Wasser re-dispergiert und anschließend hiernach mit einer Ampulle der zweiten Zubereitung B5 und 50 ml Glukoselösung (Glukosekonzentration: 5 Gew.%) vermischt.

### Vergleichsbeispiele C1 bis C4

Die nachfolgenden Vergleichsbeispiele C1 bis C4 betreffen wie die zuvor beschriebenen Ausführungsbeispiele B1 bis B5 solche Zubereitungen, die für die parenterale Anwendung bestimmt sind und die in einer ersten Zubereitung unterschiedliche zytostatisch wirkende Wirkstoffe und in einer zweiten Zubereitung unterschiedliche biologischen Elektronenakzeptoren enthalten, wobei die erste Zubereitung mit der zweiten Zubereitung erst unmittelbar vor dem Gebrauch vermischt wird.

Zur Herstellung der ersten Zubereitungen C1 bis C4, die den zytostatisch wirkenden Wirkstoff enthalten, wurden die in der Tabelle 5 genannten Bestandteile in den dort genannten Mengen jeweils in Ethanol gelöst. Nach Abzug des Ethanols unter Vakuum und Inertgas wurde der verbliebene Rückstand in Wasser dispergiert, wobei bei der Zubereitung C1 20 l Wasser und bei den Zubereitungen C2 bis C4 jeweils 10 l Wasser verwendet wurden. Anschließend erfolgte eine Homogenisierung der so hergestellten Dispersion.

Hiernach wurde der homogenisierten Dispersion eine wäßrige Maltoselösung zugesetzt, wobei bei der ersten Zubereitung C1 diese Maltoselösung 2 kg Maltose und 2 l Wasser und bei den Zubereitungen C2 bis C4 diese Maltoselösung jeweils 1 kg Maltose und 1 l Wasser aufwies.

Nach homogener Mischung wurde die so entstandene Dispersion unter Verwendung eines 0,2 µm Filters steril filtriert.

Nach Abfüllung von 100 mg Flavopiridol in 20 ml Vials (C1), von 10 mg Daunorubicin in 10 ml Vials (C2), von 10 mg Idarubicin in 10 ml Vials (C3) und von 10 mg Doxorubicin in 10 ml Vials (C4) wurden die diesbezüglichen Vials gefriergetrocknet.

Zur Herstellung der zweiten Zubereitungen C1 bis C4, die unterschiedliche biologische Elektronenakzeptoren enthalten, wurden die in Tabelle 6 genannten Bestandteile in den dort genannten Mengen jeweils in Wasser gelöst. Hiernach wurde die diesbezügliche Lösung unter Verwendung eines 0,2 µm Filters steril filtriert und in Ampullen abgefüllt, wobei diese Ampullen 250 mg Betainhydrogencitrat in 5 ml (C1), 100 mg Cholincitrat in 5 ml (C2), 250 mg Cholincitrat in 5 ml (C3) und 250 mg Betainhydrogencitrat in 5 ml (C4) aufwiesen.

**Tabelle 5**

| Zusammensetzung der ersten, den zytostatisch wirkenden Wirkstoff enthaltenden Zubereitungen | | | | |
|---|---|---|---|---|
| | C1 | C2 | C3 | C4 |
| Flavopiridol-HCl | 100 g | - | - | - |
| Phosphatidylcholin | 2000 g | 1000 g | 1000 g | 1000 g |
| Daunorubicin | 100 g | 10 g | - | - |
| Doxorubicin | - | - | - | 10 g |
| Idarubicin | - | - | 10 g | - |
| DSPG | 40 g | 20 g | 20 g | 20 g |
| Ethanol | 10 l | 5 l | 5 l | 50 l |
| DSPG = Distearoylphosphatidylglycerol | | | | |

**Tabelle 6**

| Zusammensetzung der zweiten, den biologischen Elektronenakzeptor enthaltenden Zubereitungen C1 bis C4 | | | | |
|---|---|---|---|---|
| | C1 | C2 | C3 | C4 |
| Betaindihydrogencitrat | 250 g | - | - | 250 g |
| Cholincitrat | - | 100 g | 250 g | - |
| Wasser | 5 l | 5 l | 5 l | 5 l |

Unmittelbar vor der Infusion wurde der jeweilige trockene, in Vials aufbewahrte zytostatische Wirkstoff in Wasser re-dispergiert, wobei beim Ausführungsbeispiel C1 20 ml Wasser und bei den Ausführungsbeispielen C2 bis C4 jeweils 10 ml Wasser hierfür verwendet wurden. Hiernach erfolgte ein gründliches Vermischen der redispergierten Wirkstoffe mit den zuvor beschriebenen zweiten Zubereitungen C1 bis C4, die in entsprechenden Ampullen aufbewahrt wurden. Weiterhin wurden jeweils 250 ml Glukoselösung (Glukosekonzentration: 5 Gew.%) zugesetzt und mit den zuvor aufgeführten beiden Flüssigkeiten vermischt.

### Vergleichsbeispiele D1 bis D4

Die nachfolgenden Vergleichsbeispiele D1 bis D4 betreffen solche Zubereitungen, die für die orale Anwendung bestimmt sind und die gleichzeitig unterschiedliche zytostatisch wirkende Wirkstoffe sowie unterschiedliche biologische Elektronenakzeptoren enthalten.

Zur Herstellung der in den Ausführungsbeispielen D1 bis D3 beschriebenen Sachets wurden die in der Tabelle 7 aufgeführten Bestandteile in einer entsprechenden Mischeinrichtung homogen miteinander vermischt.

Hiernach wurde das entsprechende Sachet zu 100 mg abgefüllt, so daß dementsprechend eine Idarubicin-Konzentration von 25 mg/Sachet resultierte. Zur Anwendung einer derartigen oral einzunehmenden Zubereitung ist es dann lediglich erforderlich, das Sachet gemäß der Zusammensetzung D1 bis D3 in einem Glas Wasser zu dispergieren.

**Tabelle 7**

| Bestandteile der oral anzuwendenden Zubereitungen D1 bis D3 | | | |
|---|---|---|---|
| | D1 | D2 | D3 |
| Idarubicin | 25 kg | 25 kg | 25 kg |
| Betaindihydrogencitrat | 500 kg | - | - |
| Cholincitrat | - | 500 kg | 500 kg |
| Sorbitol | 200 kg | 200 kg | 200 kg |
| Mannitol | 250 kg | 250 kg | 250 kg |
| Natriumcyclamat | 10 kg | 10 kg | 10 kg |
| Citronenaroma | 15 kg | 15 kg | 15 kg |

Die oral anzuwendende Zubereitung D4 wurde wie folgt hergestellt:

25 kg Idarubicin wurden mit 100 kg mikrokristallinerCellulose und 5 kg Magnesiumstearat homogen gemischt. Von dieser Mischung wurden 130 mg in Hartgelatinekapseln abgefüllt, was einer Konzentration von 100 mg Idarubicin entsprach. Eine zweite Zubereitung wurde dadurch erstellt, daß 200 mg Betainhydrogencitrat, 50 kg Sorbitol und 2,5 kg Saccharin Natrium in 5000 l Wasser gelöst wurden. Nach einer Sterilfiltration unter Verwendung eines 0,2 µm Filters wurde diese Lösung in 5 ml Trinkampullen abgefüllt, wobei jede Trinkampulle eine Konzentration an biologischem Elektronenakzeptor von 200 mg aufwies.

Zur Anwendung wurde das Ausführungsbeispiel D4 derart dargereicht, daß der jeweilige Patient eine Hartgelatinekapsel, die den zytostatisch wirkenden Wirkstoff enthielt, zusammen mit dem Inhalt einer Trinkampulle eingenommen hat.

Das bei den Ausführungsbeispielen zur Herstellung derselben verwendete Wasser stellt Wasser für Injektionen (W.f.I.) dar.

## Patentansprüche

1. Zubereitung enthaltend mindestens einen zytostatisch wirkenden Wirkstoff, wenigstens einen biologischen Elektronenakzeptor der allgemeinen Formel A 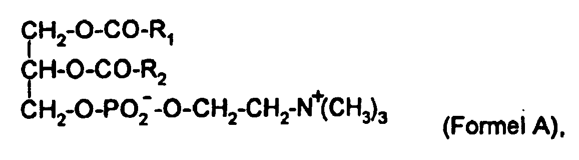 wobei R₁ und R₂ unabhängig voneinander Wasserstoff oder den Rest einer gesättigten oder ungesättigten C₁-C₂₂-Fettsäure bedeuten, und wobei der zytostatisch wirkende Wirkstoff ein Wirkstoff auf der Basis von 4H-1-Benzopyran-4-on, deren Derivate und/oder deren Salze ist sowie pharmazeutisch übliche Zusätze.

2. Zubereitung nach Anspruch 1, **dadurch gekennzeichnet, daß** R₁ und R₂ Palmitin-, Stearin-, Öl-, Linol- oder Linolensäure bedeuten.

3. Zubereitung nach einem der Ansprüche 1 bis 2, **dadurch gekennzeichnet, daß** die Zubereitung als biologischen Elektronenakzeptor Glycerophosphocholin, Phosphatidylcholin, Mischungen und/oder Derivate davon enthält.

4. Zubereitung nach einem der Ansprüche 1 bis 3, **dadurch gekennzeichnet, daß** das molare Massenverhältnis des biologischen Elektronenakzeptors zum zytostatisch wirkenden Wirkstoff zwischen 0,1:1 und 5:1, vorzugsweise zwischen 0,5:1 und 2:1, variiert.

5. Zubereitung nach einem der Ansprüche 1 bis 4, **dadurch gekennzeichnet, daß** die Zubereitung als biologischen Elektronenakzeptor ein Phospholipid, insbesondere ein pflanzliches Phospholipid und vorzugsweise Soja-Phospholipid, aufweist.

6. Zubereitung nach Anspruch 5, **dadurch gekennzeichnet, daß** das Phospholipid eine Konzentration an Phosphatidytcholin von wenigstens 50 Gew.%, bezogen auf die Gesamtmenge des in der Zubereitung enthaltenen phospholipidischen biologischen Elektronenakzeptors, enthält.

7. Zubereitung nach einem der Ansprüche 5 bis 6, **dadurch gekennzeichnet, daß** die Konzentration des Phosphatidylcholins größer als 70 Gew.%, vorzugsweise größer als 80 Gew.% und insbesondere größer als 90 Gew.%, jeweils bezogen auf die Gesamtmenge des in der Zubereitung enthaltenen phospholipidischen biologischen Elektronenakzeptors, ist.

8. Zubereitung nach einem der Ansprüche 5 bis 7, **dadurch gekennzeichnet, daß** der phospholipidische biologische Elektronenakzeptor eine Mischung von Phospholipiden darstellt, wobei die Mischung neben Phosphatidylcholin mindestens noch ein negativ geladenes Phospholipid, insbesondere Phosphatidsäure, aufweist.

9. Zubereitung nach Anspruch 8, **dadurch gekennzeichnet, daß** das negativ geladene Phospholipid in der Phospholipidmischung in einer Konzentration zwischen 2 Gew.% und 10 Gew.%, bezogen auf die Gesamtmenge des in der Zubereitung enthaltenen phospholipidischen biologischen Elektronenakzeptors, vorliegt.

10. Zubereitung nach einem der Ansprüche 1 bis 9, **dadurch gekennzeichnet, daß** die Zubereitung als Injektions- oder Infusionsflüssigkeit formuliert ist, daß die Zubereitung als zytostatisch wirkenden Wirkstoff Flavopiridol-HCl aufweist und daß die Zubereitung als biologischen Elektronenakzeptor Phosphatidylcholin enthält.

11. Zubereitung nach Anspruch 10, **dadurch gekennzeichnet, daß** die Konzentration des zytostatischen Wirkstoffes zwischen 1 mg und 200 mg, insbesondere zwischen 5 mg und 40 mg, variiert.

12. Zubereitung nach einem der Ansprüche 9 bis 10, **dadurch gekennzeichnet, daß** die Konzentration an biologischem Elektronenakzeptor zwischen 50 mg und 3 mg, insbesondere zwischen 250 mg und 1 mg, variiert.

13. Zubereitung nach einem der Ansprüche 1 bis 9, **dadurch gekennzeichnet, daß** die Zubereitung als oral darzureichende Zubereitung formuliert ist, daß die Zubereitung als zytostatisch wirkenden Wirkstoff Flavopiridol-HCl aufweist und daß die Zubereitung als biologischen Elektronenakzeptor Phosphatidylcholin enthält.

14. Zubereitung nach Anspruch 13, **dadurch gekennzeichnet, daß** die Konzentration des zytostatischen Wirkstoffes zwischen 5 mg und 70 mg, insbesondere zwischen 15 mg und 40 mg, variiert.

15. Zubereitung nach einem der Ansprüche 13 bis 14, **dadurch gekennzeichnet, daß** die Konzentration an biologischem Elektronenakzeptor zwischen 50 mg und 3 mg, insbesondere zwischen 250 mg und 1 mg, variiert.

16. Zubereitung nach einem der Ansprüche 1 bis 15, **dadurch gekennzeichnet, daß** die Zubereitung zwei flüssige oder feste Zubereitungen oder eine feste und eine flüssige Zubereitung umfaßt, wobei eine erste Zubereitung mindestens einen zytostatisch wirkenden Wirkstoff auf der Basis von 4H-1-Benzopyran-4-on, deren Derivate und/oder deren Salze und eine zweite Zubereitung mindestens einen biologischen Elektronenakzeptor enthält.

17. Verwendung einer Zubereitung gemäß einem der Ansprüche 1 bis 16, zur Herstellung eines Medikamentes zur Behandlung von Tumoren, insbesondere zur Behandlung von Krebs.

18. Verwendung der Zubereitung gemäß Anspruch 17, wobei die tägliche Dosis des zytostatischen Wirkstoffes von 0,0001 g bis 2 g, insbesondere von 0,01 g bis 1 g ist, die Dosis des biologischen Elektronenakzeptors von 0,1 g bis 100 g, insbesondere von 5 g bis 50 g ist, jeweils bezogen auf einen Quadratmeter der Körperoberfläche des zu behandelnden Patienten.

## Claims

1. A preparation comprising at least one compound having cytostatic activity, at least one biological electron acceptor of the formula A, 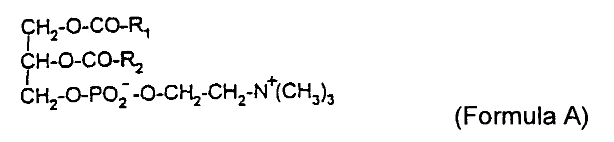 where R₁ and R₂ are, independently of one another, hydrogen or the radical of a saturated or unsaturated C₁-C₂₂-fatty acid, and wherein the active compound having cytostatic activity is an active compound based on 4H-1-benzopyran-4-one, their derivatives and/or their salts, and also pharmaceutically customary additives.

2. The preparation as claimed in claim 1, **characterized in that** R₁ and R₂ are palmitic, stearic, oleic, linoleic or linolenic acid.

3. The preparation as claimed in one of claims 1 to 2, **characterized in that** the preparation contains as biological electron acceptor glycero-phosphocholine, phosphatidylcholine, mixtures and/or derivatives thereof.

4. The preparation as claimed in one of claims 1 to 3, **characterized in that** the molar mass ratio of the biological electron acceptor to the active compound having cytostatic activity varies between 0.1:1 and 5:1, preferably between 0.5:1 and 2:1.

5. The preparation as claimed in one of claims 1 to 4, **characterized in that** the preparation contains as biological electron acceptor a phospholipid, in particular a plant phospholipid and preferably soybean phospholipid.

6. The preparation as claimed in claim 5, **characterized in that** the phospholipid contains a concentration of phosphatidylcholine of at least 50% by weight, based on the total amount of the phospholipid biological electron acceptor contained in the preparation.

7. The preparation as claimed in one of claims 5 to 6, **characterized in that** the concentration of the phosphatidylcholine is greater than 70% by weight, preferably greater than 80% by weight and in particular greater than 90% by weight, in each case based on the total amount of the phospholipid biological electron acceptor contained in the preparation.

8. The preparation as claimed in one of claims 5 to 7, **characterized in that** the phospholipid biological electron acceptor is a mixture of phospholipids, the mixture containing, in addition to phosphatidylcholine, also at least one negatively charged phospholipid, in particular phosphatidic acid.

9. The preparation as claimed in claim 8, **characterized in that** the negatively charged phospholipid in the phospholipid mixture is present in a concentration of between 2% by weight and 10% by weight, based on the total amount of the phospholipid biological electron acceptor contained in the preparation.

10. The preparation as claimed in one of claims 1 to 9, **characterized in that** the preparation is formulated as an injection or infusion fluid, **in that** the preparation contains as active compound having cytostatic activity flavopiridol HCl and **in that** the preparation contains as biological electron acceptor phosphatidylcholine.

11. The preparation as claimed in claim 10, **characterized in that** the concentration of the cytostatic active compound varies between 1 mg and 200 mg, in particular between 5 mg and 40 mg.

12. The preparation as claimed in one of claims 9 to 10, **characterized in that** the concentration of biological electron acceptor varies between 50 mg and 3 mg, in particular between 250 mg and 1 mg.

13. The preparation as claimed in one of claims 1 to 9, **characterized in that** the preparation is formulated as a preparation for oral administration, **in that** the preparation contains as active compound having cytostatic activity flavopiridol HCl and **in that** the preparation contains as biological electron acceptor phosphatidylcholine.

14. The preparation as claimed in claim 13, **characterized in that** the concentration of the cytostatic active compound varies between 5 mg and 70 mg, in particular between 15 mg and 40 mg.

15. The preparation as claimed in one of claims 13 to 14, **characterized in that** the concentration of biological electron acceptor varies between 50 mg and 3 mg, in particular between 250 mg and 1 mg.

16. The preparation as claimed in one of claims 1 to 15, **characterized in that** the preparation comprises two liquid or solid preparations or one solid and one liquid preparation, a first preparation containing at least one active compound having cytostatic activity based on 4H-1-benzopyran-4-one, their derivatives and/or their salts and a second preparation containing at least one biological electron acceptor.

17. The use of a preparation as claimed in one of claims 1 to 16 for the production of a medicament for the treatment of tumors, in particular for the treatment of cancer.

18. The use of the preparation as claimed in claim 17, the daily dose of the cytostatic active compound being from 0.0001 g to 2 g, in particular from 0.01 g to 1 g, the dose of the biological electron acceptor being from 0.1 g to 100 g, in particular from 5 g to 50 g, in each case based on a square meter of the body surface of the patient to be treated.

## Revendications

1. Préparation contenant au moins une substance active à effet cytostatique, au moins un accepteur biologique d'électrons de formule générale A 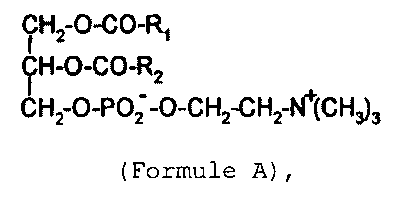 dans laquelle R₁ et R₂ représentent, indépendamment l'un de l'autre, hydrogène ou le résidu d'un acide gras en C₁ à C₂₂ saturé ou insaturé, et dans laquelle la substance active à effet cytostatique est une substance active à base de 4H-1-benzopyrann-4-one, ses dérivés et/ou ses sels, ainsi que les additifs pharmaceutiques usuels.

2. Préparation selon la revendication 1, **caractérisée en ce que** R₁ et R₂ signifient l'acide palmitique, stéarique, oléique, linoléique ou linolénique.

3. Préparation selon l'une quelconque des revendications 1 à 2, **caractérisée en ce que** la préparation contient, comme accepteur biologique d'électrons, de la glycérophosphocholine, de la phosphatidylcholine, des mélanges et/ou des dérivés de ceux-ci.

4. Préparation selon l'une quelconque des revendications 1 à 3, **caractérisée en ce que** le rapport de masses molaires de l'accepteur biologique d'électrons à substance active à effet cytostatique varie entre 0,1:1 et 5:1, de préférence entre 0,5:1 et 2:1.

5. Préparation selon l'une quelconque des revendications 1 à 4, **caractérisée en ce que** la préparation contient, comme accepteur biologique d'électrons, un phospholipide, en particulier un phospholipide végétal et de préférence un phospholipide de soja.

6. Préparation selon la revendication 5, **caractérisée en ce que** le phospholipide contient une concentration en phosphatidylcholine d'au moins 50% en poids, par rapport à la quantité totale de l'accepteur biologique d'électrons phospholipidique contenu dans la préparation.

7. Préparation selon l'une quelconque des revendications 5 à 6, **caractérisée en ce que** la concentration en phosphatidylcholine est supérieure à 70% en poids, de préférence supérieure à 80% en poids et en particulier supérieure à 90% en poids, à chaque fois par rapport à la quantité totale de l'accepteur biologique d'électrons phospholipidique contenu dans la préparation.

8. Préparation selon l'une quelconque des revendications 5 à 7, **caractérisée en ce que** l'accepteur biologique d'électrons phospholipidique est un mélange de phospholipides, le mélange présentant, outre la phosphatidylcholine, au moins encore un phospholipide chargé négativement, en particulier l'acide phosphatidique.

9. Préparation selon la revendication 8, **caractérisée en ce que** le phospholipide chargé négativement se trouve dans le mélange phospholipidique en une concentration entre 2% en poids et 10% en poids, par rapport à la quantité totale de l'accepteur biologique d'électrons phospholipidique contenu dans la préparation.

10. Préparation selon l'une quelconque des revendications 1 à 9, **caractérisée en ce que** la préparation est formulée en tant que liquide destiné à l'injection ou à l'infusion, **en ce que** la préparation présente comme substance active à effet cytostatique le Flavopiridol-HCl et **en ce que** la préparation contient comme accepteur biologique d'électrons la phosphatidylcholine.

11. Préparation selon la revendication 10, **caractérisée en ce que** la concentration en substance active cytostatique varie entre 1 mg et 200 mg, en particulier entre 5 mg et 40 mg.

12. Préparation selon l'une quelconque des revendications 9 à 10, **caractérisée en ce que** la concentration en accepteur biologique d'électrons varie entre 50 mg et 3 mg, en particulier entre 250 mg et 1 mg.

13. Préparation selon l'une quelconque des revendications 1 à 9, **caractérisée en ce que** la préparation est formulée en tant que préparation à administrer par voie orale, **en ce que** la préparation présente comme substance active à effet cytostatique le Flavopiridol-HCl et **en ce que** la préparation présente comme accepteur biologique d'électrons la phosphatidylcholine.

14. Préparation selon la revendication 13, **caractérisée en ce que** la concentration en substance active cytostatique varie entre 5 mg et 70 mg, en particulier entre 15 mg et 40 mg.

15. Préparation selon l'une quelconque des revendications 13 à 14, **caractérisée en ce que** la concentration en accepteur biologique d'électrons varie entre 50 mg et 3 mg, en particulier entre 250 mg et 1 mg.

16. Préparation selon l'une quelconque des revendications 1 à 15, **caractérisée en ce que** la préparation comprend deux compositions liquides ou solides ou une préparation solide et une préparation liquide, une première préparation contenant au moins une substance active à effet cytostatique à base de 4H-1-benzopyrann-4-one, ses dérivés et/ou ses sels et une deuxième préparation contenant au moins un accepteur biologique d'électrons.

17. Utilisation d'une préparation selon l'une quelconque des revendications 1 à 16 pour la préparation d'un médicament destiné au traitement de tumeurs, en particulier au traitement du cancer.

18. Utilisation de la préparation selon la revendication 18, la dose journalière de la substance active cytostatique étant de 0,0001 g à 2 g, en particulier de 0,01 g à 1 g, la dose de l'accepteur biologique d'électrons est de 0,1 g à 100 g, en particulier de 5 g à 50 g, à chaque fois par rapport à un mètre carré de la surface corporelle du patient à traiter.
